# EUROPEAN PATENT APPLICATION

(11) **EP 2 036 519 A1**
(43) Date of publication of application: **18.03.2009**
(21) Application number: 07018170.6
(22) Date of filing: 16.09.2007
(51) Int. Cl.: A61F 2/84

(54) **Bifurcation stent and stent delivery assembly**

(71) Applicant: Perelson, Ophir, Beverly Hills CA 90211 (US)
(72) Inventor: Perelson, Ophir, Beverly Hills CA 90211 (US)
(74) Representative: Arth, Hans-Lothar

(57) **Abstract**

The present invention relates to a novel bifurcation stent delivery assembly for treating a lesion at a bifurcation in a body and methods for the use of the novel bifurcation stent delivery assembly.

## Description

The present invention relates to a novel bifurcation stent and stent delivery assembly for treating a lesion at a bifurcation of blood vessels or other tubular structures in a body and methods for the use of the novel bifurcation stent delivery assembly.

### BACKGROUND OF THE INVENTION

A stent is a medical device designed to serve as a temporary or permanent internal scaffold to maintain or increase the lumen of a body conduit. Coronary stents were first introduced to prevent arterial dissections and to eliminate vessel recoil and intimal hyperplasia associated with percutaneous transluminal coronary angioplasty. Coronary stenting has now become an established mode of treatment in percutaneous transluminal coronary interventions and it has been shown to reduce late restenosis relative to conventional balloon angioplasty. The stent application range expanded as more experience was gained, and encouraging results have been obtained in the treatment of a range of vascular diseases. Stents are currently used for support of additional body conduits, including the urethra, trachea, and esophagus.

Despite the development and progression of stents, they continued to have limitations such as the treatment of lesions close to or at a vessel bifurcation. In this case the placement of a stent close to a bifurcation of a parent vessel to form a first branch vessel and a second branch vessel can cause the obstruction or distortion of one of the branch vessels. To overcome this limitation the placement of a stent in each of the parent, first branch and second branch vessels is possible. However the accurate placement of multiple separate stents is a complicated procedure, and once in place the multiple disruptions to the blood flow and the vessel wall caused by the ends of the separate stents present an increased risk of stenosis.

As a result, bifurcated (branched) stents were developed. The bifurcated stent is a single stent structure having a parent body and a first branch arm and second branch arm connected to the parent body. However the delivery and accurate placement of the branched stent presented new challenges in stent delivery. The delivery of a single stent structure having a first branch arm and a second branch arm into a first branch vessel and a second branch vessel presented new challenges in stent delivery.

WO 01/43665 presents one possible solution to the problem of delivering a bifurcated stent. In W0 01/43665 a bifurcated stent having a first and second branch arm is carried on a bifurcated balloon catheter having a first and second balloon arm wherein the first and second branch arms of the stent are carried on the first and second balloon arms of the catheter respectively. For delivery the bifurcated stent carried on the bifurcated balloon catheter is carried inside a single sheath.

The objective of the present invention is to provide an improved stent delivery assembly for placing a bifurcated stent at a bifurcation in a vessel.

This objective is solved by the technical teaching of the independent claims of the present invention. Further advantageous embodiments of the invention result from the dependent claims, the description and the figures.

### DESCRIPTION OF THE INVENTION

The present invention is directed to stent delivery assembly for positioning a self-expanding bifurcated stent that is contained in a retractable sheath at a desired site in a patient vessel.

A bifurcated stent has by definition a parent member (parent expandable member) and two branch members (first branch expandable member and second branch expandable member). These parent and branch members form a Y shaped bifurcated stent, the parent member forming the base (trunk) of the Y and the two branch members forming the arms (branches) of the Y.

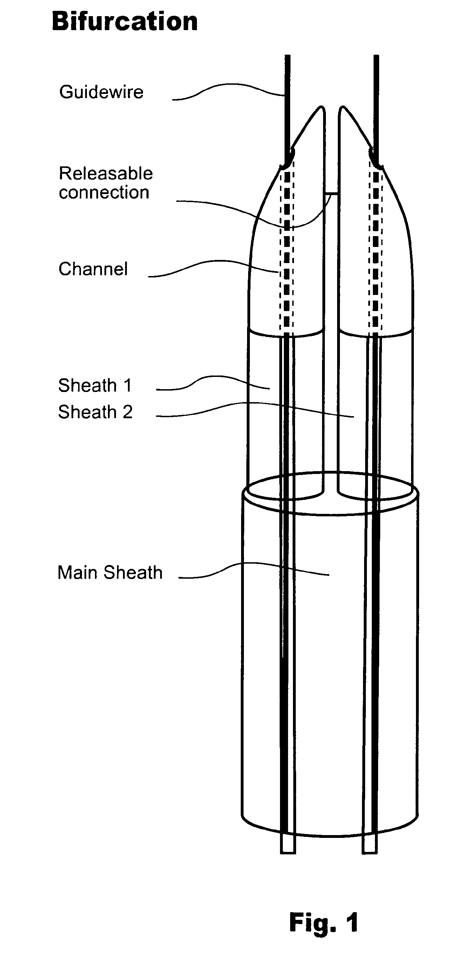

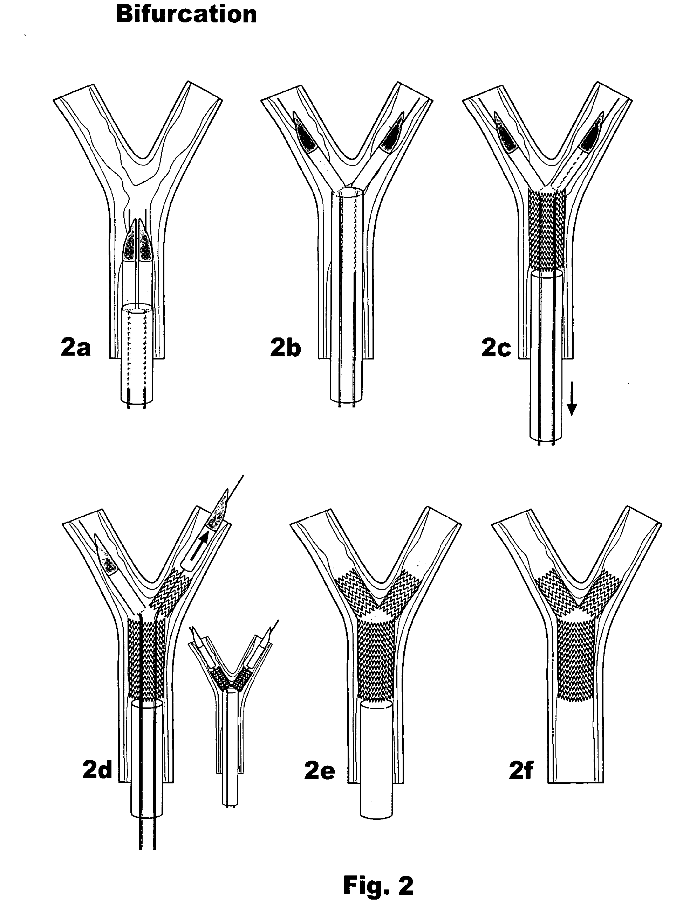

In the present invention the delivery system is constructed in such way that each of the base and the branches of the Y shaped bifurcated stent have a separate sheath, thus consisting of three parts (Fig. 1). For ease of use the first branch sheath (first branch tubular member) and the second branch sheath (second branch tubular member) are connected to each other by a releasable connection that holds the two sheath branches together during delivery to a desired site in the patient body (Fig. 2a). The two sheathes of the first and second branch are only removed after the two branches of the bifurcated stent (first branch expandable member and second branch expandable member) have been accurately positioned in the two branches of the bifurcated vessel of the patient (Fig. 2b). The base of the stent (parent expandable member) is deployed by withdrawal of the parent sheath (parent tubular member) back towards the operator (Fig. 2c). In order to deploy the branches of a bifurcated stent, the two branch sheaths are initially moved in a direction distal to the stent that is, in the direction away from the operator inserting the stent (Fig. 2d). Further the two branch sheaths are each able to be removed independently, allowing for the first branch sheath to be removed before the second branch sheath or vice versa. After the deployment of the branches of the bifurcated stent, the branch sheaths are withdrawn back through the lumen of expanded branches of the stent (Fig. 2e). After deployment of the entire stent (both branch members and parent member) the entire delivery system can be removed (Fig. 2f).The base of the stent (parent expandable member) can also be deployed after the deployment of the first and second branch expandable members, if this is desired by the operator or required by the clinical scenario.

One example of a stent delivery assembly according to the present invention includes a mechanism for controlling the movement of the parent tubular member in a proximal direction and the movement of the first branch tubular member and the second branch tubular member in a distal direction. The parent tubular member and the first branch tubular member and the second branch tubular member are distinct members that fully cover the stent in a compressed state.

The first branch tubular member and the second branch tubular member further include a channel that forms a first guidewire lumen and a second guidewire lumen, respectively. The first guidewire lumen and a second guidewire lumen can be made of plastic, metal or other suitable material. The first guidewire lumen and a second guidewire lumen is coupled at a proximal end thereof to the mechanism and at a distal end thereof to the distal tip of first branch tubular member and the second branch tubular member. The inner tubular member encloses a guidewire which aids in the navigation of the stent delivery assembly to and through the vessel to be treated. The first guidewire lumen and a second guidewire lumen can have a single central lumen for the guidewire or it may have a multichannel construction. The first guidewire lumen and a second guidewire lumen are preferably made of a flexible but incompressible construction such as a polymer encapsulated braid or coil, or a thin walled plastic or metallic tube as known in the art.

The movement of the first branch tubular member and the second branch tubular member can be controlled by the movement of the first guidewire lumen and the second guidewire lumen. The movement of the parent tubular member can be controlled by movement of the parent tubular member itself.

The parent tubular member and the first branch tubular member and the second branch tubular member are operably coupled to a mechanism such that parent tubular member and first branch tubular member and the second branch tubular member can be moved apart in a controlled manner, by operation of the mechanism, to thereby expose and deploy a bifurcated stent.

Mechanisms for controlling the movement of separate tubular members in stent delivery systems are known in the art. Any system that allows the independent movement of the parent tubular member and the first branch tubular member and the second branch tubular member is suitable for the purposes of the present invention.

Thus the present invention provides a novel delivery system for a bifurcated stent having the advantage of maintaining the branches of a bifurcated stent in a contracted state until after they are accurately placed in the desired bifurcated vessel branches and allowing the independent deployment of the branches of the bifurcated stent in the patient vessel.

The following provides a more detailed description of the stent delivery assembly and the method of use of the stent delivery assembly.

For the purpose of this description the terms "proximal" and "distal" are defined in relation to the point of entry into the patient of the stent delivery assembly. Proximal is a position closer of the point of entry of the stent delivery assembly and distal is a position further away from the point of entry of the stent delivery assembly. Thus the distal end of the stent assembly is the end of the assembly furthest away from the point of entry of the assembly.

The present invention is directed to a stent delivery assembly for treating a lesion at a bifurcation in a body, where a parent vessel divides/bifurcates into a first branch vessel and a second branch vessel. A bifurcation is defined as the point at which a single vessel divides (branches) into two separate vessels.

The stent delivery assembly of the present invention comprises a first branch tubular member and a second branch tubular member and a parent tubular member: These tubular members form the sheath that is used to enclose the stent as explained above.

The stent delivery assembly also comprises channels that form a first guidewire lumen and a second guidewire lumen, the first guidewire lumen for receiving a first guidewire and the second guidewire lumen for receiving a second guidewire. The distal end of the first guidewire lumen is connected to the distal end of the first branch tubular member and the distal end of the second guidewire lumen is connected to the distal end of the second branch tubular member. The first guidewire is extendible beyond the distal end of the first guidewire lumen and the second guidewire is extendible beyond the distal end of the second guidewire lumen.

Preferably the first guide wire lumen comprises a means for advancing the first branch tubular member and the second guidewire lumen comprises a means for advancing the second branch tubular member. In addition the the first guide wire lumen comprises a means for subsequently withdrawing the first branch tubular member and the second guidewire lumen comprises a means for subsequently withdrawing the second branch tubular member.

The first guidewire lumen and the second guidewire lumen can be moved independently of each other and the first guidewire lumen and the second guidewire lumen can also be moved simultaneously.

The stent to be delivered using the stent delivery assembly of the present invention comprises a parent expandable member, a first branch expandable member and a second branch expandable member. In reference to the Y shaped bifurcated stent described above, the parent expandable member forms the base of the Y and the first and second branch expandable members form the arms of the Y. The first branch tubular member receives the first branch expandable member, the second branch tubular member receives the second branch expandable member, and the parent tubular member receives the parent expandable member. The tubular member maintains the respective expandable member in a contracted state during delivery of the expandable member to a desired site in a patient.

In the inventive stent delivery assembly the first branch tubular member and the second branch tubular member have a releaseable connection between the first branch tubular member and the second branch tubular member. This releaseable connection between the two tubular members binds the two branch tubular members together and allows for ease of movement of the two branch tubular members within a single lumen until reaching the bifurcation point in a vessel.

The releasable connection between the two branch tubular members is able to be broken when the two tubular member branches reach the bifurcation due to the pressure exerted by the operator inserting the stent delivery assembly. As the first branch tubular member is inserted into a first branch vessel and the second branch tubular member is inserted into a second branch vessel the pressure exerted by the operator forces the first and second branches apart, to either side of the bifurcation, breaking the releasable connection. In addition to this, the bifurcation itself can also exert pressure on the releasable connection, if the connection comes into direct contact with the bifurcation before breaking, and also cause the releasable connection to break.

Preferably each of the first branch tubular member, the second branch tubular member and the parent tubular member form a separate sheath.

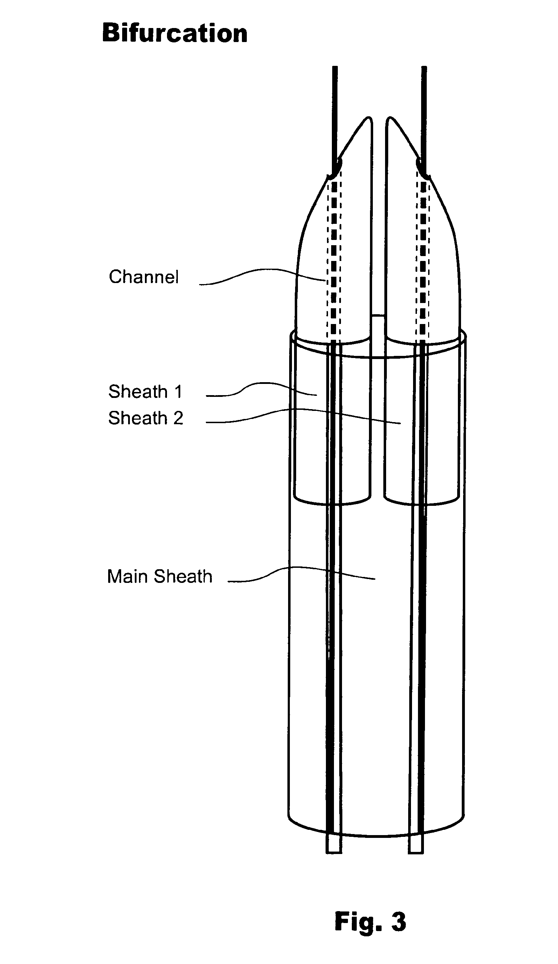

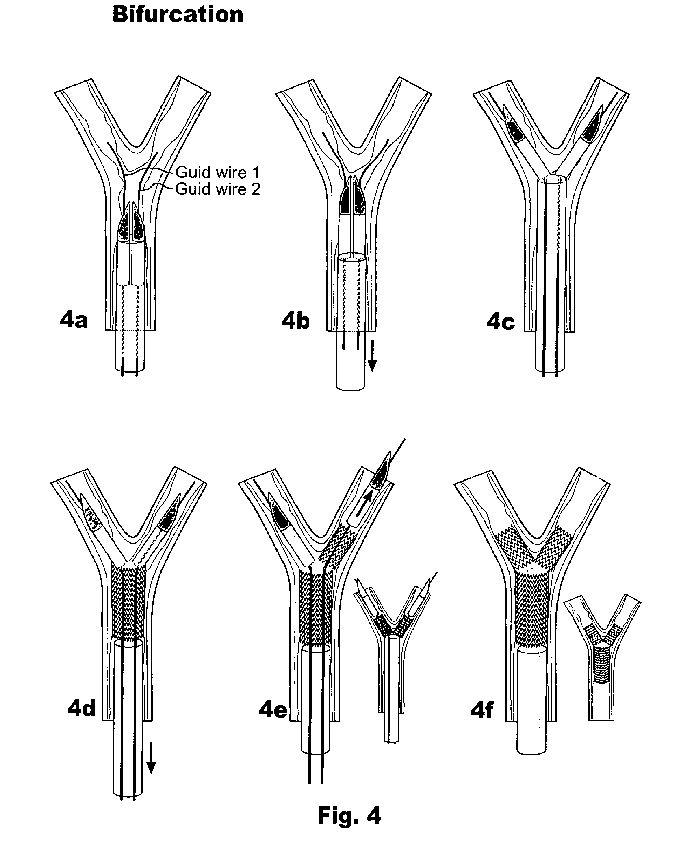

It is also possible for the first branch tubular member and the second branch tubular member to both be contained in the parent tubular member (Fig. 3) and for parent tubular member to be retractable from the first branch tubular member and the second branch tubular member just prior to the branch tubular members entering the branch vessels at the bifurcation (Fig. 4a,b). In this case no releasable connection between the first and the second branch tubular members is needed. All other stages of stent delivery are the same as for the first embodiment (Fig. 4c-f).

Preferably the first branch expandable member and the second branch expandable member are connected to the parent expandable member to form a bifurcated stent

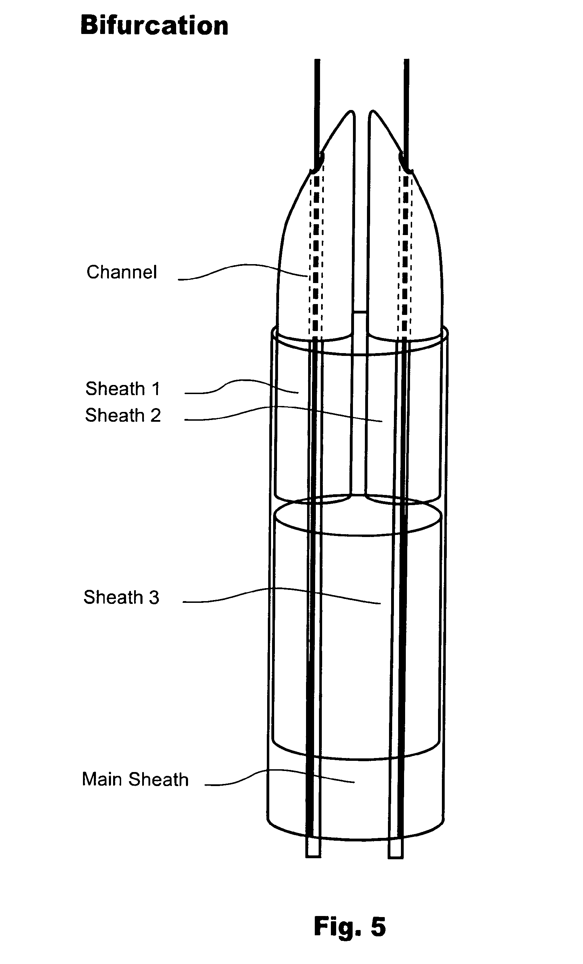

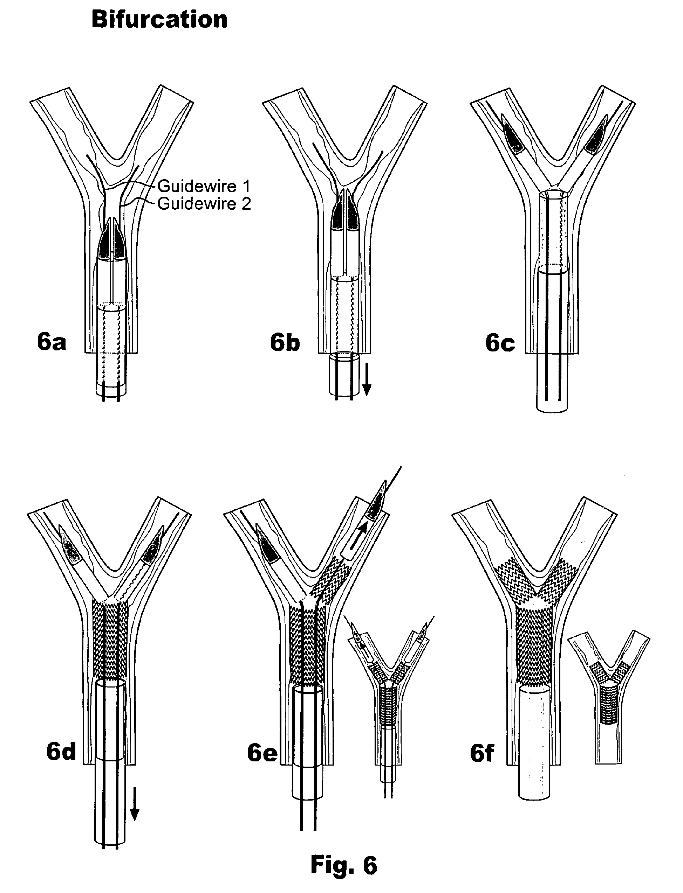

Further, the first branch tubular member and the second branch tubular member and the parent tubular member may all be contained in an outer tubular member (Fig. 5). The outer tubular member would provide a lumen for delivery of the inventive stent delivery assembly. As the first step of this embodiment delivery and deployment, the outer sheath is withdrawn back prior to the side branches of the delivery system entering the branch vessels (Fig. 6a,b). The other stages of the stent deployment are similar to the first and second embodiments (Fig. 6c-f).

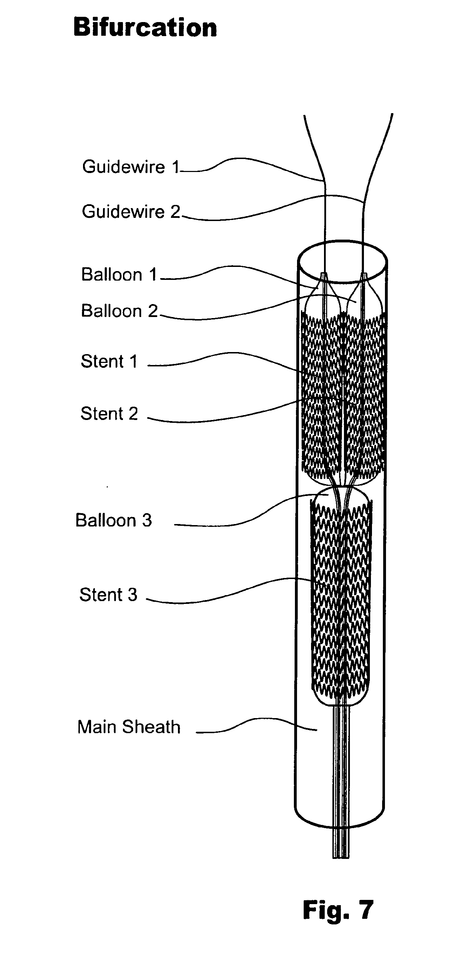

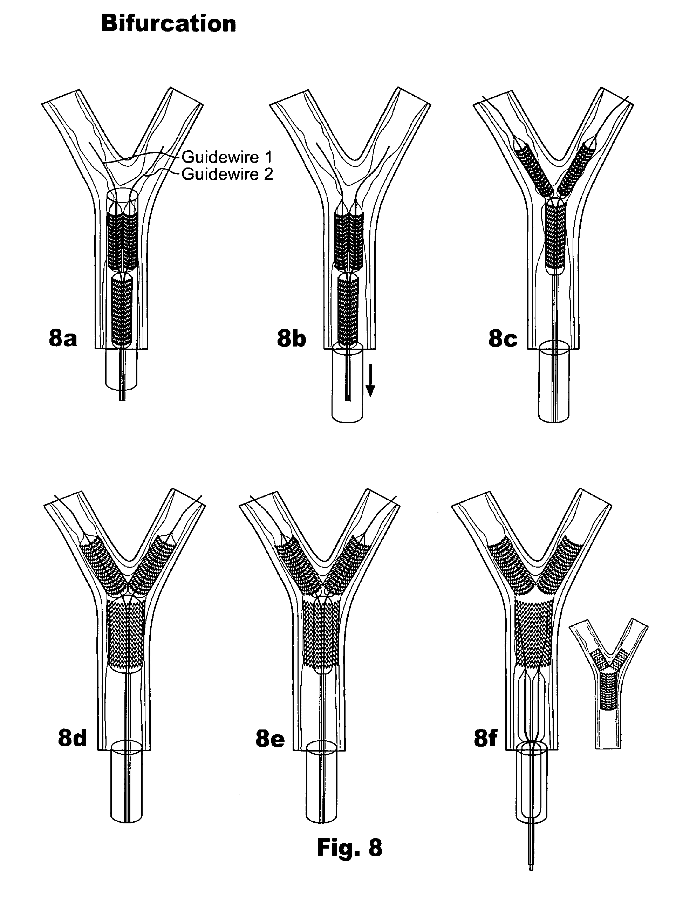

Alternatively the stent delivery assembly can comprise a bifurcated balloon catheter having a parent balloon segment, a first branch balloon segment and a second branch balloon segment, wherein the parent expandable member is carried on the parent balloon segment, the first branch expandable member is carried on the first branch balloon segment and the second branch expandable member is carried on the second branch balloon segment. The parent balloon segment with mounted parent balloon-expandable member, and the first and second branch balloon segments with respectively mounted first and second balloon expandable members can be covered with a single tubular member (Fig. 7). In this embodiment the expandable members can be expanded by inflating the balloon catheter after the removal of the tubular member (Fig. 8a-f).

The present invention is also directed to a method for stenting a bifurcation in a body, the bifurcation bifurcating a parent vessel into a first branch vessel and a second branch vessel using the stent delivery assembly described above.

The method of the invention and stages of a bifurcated stent deployment are shown in the Fig. 2a-f. The method of the invention comprises providing a stent delivery assembly comprising:
a first branch tubular member and a second branch tubular member and a parent branch tubular member, the first branch tubular member and the second branch tubular member having a releasable connection between the first branch tubular member and the second branch tubular member, and
a first guidewire lumen and a second guidewire lumen, the first guidewire lumen for receiving a first guidewire and the second guidewire lumen for receiving a second guidewire, the distal end of the first guidewire lumen being connected to the distal end of the first branch tubular member and the distal end of the second guidewire lumen being connected to the distal end of the second branch tubular member (Fig. 1),
wherein the stent delivery assembly receives a first branch expandable member in the first branch tubular member, a second branch expandable member in the second branch tubular member and a parent expandable member in the parent tubular member (Fig. 2a).

The stent delivery assembly is advanced along a patient vessel so that the stent delivery assembly is positioned in the parent vessel proximal of the bifurcation (Fig 2a). The first guidewire is then advanced into the first branched vessel and the second guidewire is advanced into the second branched vessel.

The stent delivery assembly is advanced until the releasable connection between the first branch tubular member and the second branch tubular member is released by the bifurcation, as explained above

After the releasable connection is released the stent delivery assembly can be further advanced so that the first branch expandable member contained in the first branch tubular member enters the first branched vessel and the second branch expandable member contained in the second branch tubular member enters the second branched vessel (Fig. 2b).

The first branch expandable member is released in the first branch vessel by advancing the first branch tubular member distally of the first expandable member and the second branch expandable member is released in the second branch vessel by advancing the second branch tubular member distally of the first expandable member (Fig. 2d). The parent expandable member is released in the parent vessel by withdrawing the parent tubular member proximally of the parent expandable member (Fig. 2c). Once all of the expandable members have been released and fully expanded by the nature of the material of the stent or stent design, the stent delivery assembly can be withdrawn from the patient.

The first and second branch tubular members are withdrawn through the lumens of fully expanded first and second expandable members, respectively, and through the lumen of fully expanded parent branch expandable member (Fig. 2e).

The first guide wire lumen comprises a means for advancing the first branch tubular member and the second guidewire lumen comprises a means for advancing the second branch tubular member. In addition the first guide wire lumen comprises a means for subsequently withdrawing the first branch tubular member and the second guidewire lumen comprises a means for subsequently withdrawing the second branch tubular member. The first guidewire lumen and the second guidewire lumen can be moved independently of each other, however the first guidewire lumen and the second guidewire lumen can also be moved simultaneously.

In the method of the invention the first branch expandable member contained in a first branch tubular member and the second branch expandable member contained in a second branch tubular member can both be contained in the parent tubular member, and further the parent tubular member can be retractable from the first branch tubular member and the second branch tubular member prior to advancing a first branch tubular member into a first branch vessel and the second branch tubular member into a second branch vessel.

When the first branch tubular member and the second branch tubular member are covered by the parent tubular member (Fig. 3) and the stages of this embodiment assembly are demonstrated in Fig. 4 a-f. In this case the delivery system is parked at the bifurcation of a vessel prior to entering the branch vessels (Fig. 4a), the parent tubular member is retracted back exposing the first and second branch tubular members, but still covering the parent expandable member (Fig. 4b). The entire delivery system is then advanced forward with the first and second branch tubular members entering respective first and second branch vessels (Fig. 4c). At this point the first and second branch expandable members can be deployed by sequentially or simultaneously advancing forward the first and second branch tubular members (Fig. 4e), followed by deployment of the parent expandable member by withdrawal of the parent tubular member (Fig. 4d). If desired by an operator the parent expandable member can be deployed prior to deployment of the first and second branch expandable members. Once again the first and second branch tubular members are withdrawn through the lumens of fully expanded first and second expandable members, respectively, and through the lumen of fully expanded parent branch expandable member (Fig 4f).

Preferably the stent for use in the method of the invention comprises a first branch expandable member and a second branch expandable member which are connected to a parent expandable member to form a self-expanding or balloon-expandable bifurcated stent.

Further, the first branch tubular member and the second branch tubular member and the parent tubular member may all be contained in an outer tubular member (Fig. 5). In this case the outer tubular member would provide a lumen for delivery of the inventive stent delivery assembly to the bifurcation.

In addition the method of the present invention can also be performed by providing a bifurcated balloon catheter having a parent balloon segment for carrying the parent expandable member, a first branch balloon segment for carrying the first branch expandable member and a second branch balloon segment for carrying the second branch expandable member, and after releasing the first branch expandable member, the second branch expandable member and the parent expandable member inflating the ballon catheter segments to expand the first branch expandable member, the second branch expandable member and the parent expandable member.

The parent balloon segment with mounted parent balloon-expandable member, and the first and second branch balloon segments with respectively mounted first and second balloon expandable members can be covered with a single tubular member (Fig. 7). The stages of a bifurcated stent delivery and deployment are shown in the Fig. 8a-f. In this case, the assembled delivery system is introduced to the bifurcation, but not entering the branch vessels (Fig. 8a), then the single sheath is withdrawn back exposing parent and first and second branch balloon expandable members mounted on respective parent and first and second branch balloon segments (Fig. 8b). The system is then advanced forward positioning the first and second branch balloon segments in the respective first and second branch vessels (Fig. 8c). The parent, first and second branch balloon-expandable members are then deployed by sequential or simultaneous inflation of the respective balloon segments (Fig. 8d). The balloon segments are then deflated and removed (Fig. 8 e,f), but the bifurcated balloon-expandable stent remains in place.

The stent delivery assembly of the present invention and the method for stenting a bifurcation in a body of the present invention is useful for prophylaxis and treatment of arteriosclerosis, atherosclerosis, arteriovenous malformations, stenosis, in-stent stenosis, restenosis which means recurrent stenosis, vascular occlusion and hyperproliferative vascular disease. Furthermore, the stent delivery assembly of the present invention and the method for stenting a bifurcation in a body of the present invention is useful for the treatment of vascular injuries which are caused or induced by mechanically mediated vascular injury, vascular catheterization, vascular scraping, percutaneous transluminal coronary angioplasty or stenting, vascular surgery and vascular laser treatment. Furthermore, the stent delivery system of the present invention and the method for stenting can be used in body tubular structures, other than blood vessels.

The stents for use in the stent delivery assembly can be any type of expandable stent known in the art and can be manufactured from any material suitable for the manufacture of an expandable stent as known in the art. The stent can be coated or uncoated and can be a stent carrying an active agent such as an antirestenosis drug. The stent delivery system can be manufactured of any material known in the art for the manufacture of stent delivery systems.

## Claims

1. A stent delivery assembly for treating a lesion at a bifurcation in a body, the bifurcation bifurcating a parent vessel into a first branch vessel and a second branch vessel, the stent delivery assembly comprising:
a first branch tubular member and a second branch tubular member and a parent tubular member,
a first guidewire lumen and a second guidewire lumen, the first guidewire lumen for receiving a first guidewire and the second guidewire lumen for receiving a second guidewire,
the distal end of the first guidewire lumen being connected to the distal end of the first branch tubular member and the distal end of the second guidewire lumen being connected to the distal end of the second branch tubular member, and wherein
the stent delivery assembly receives a first branch expandable member in the first branch tubular member, a second branch expandable member in the second branch tubular member and a parent expandable member in the parent tubular member.

2. The stent delivery assembly of claim 1 wherein the first branch tubular member and the second branch tubular member have a releaseable connection between the first branch tubular member and the second branch tubular member.

3. The stent delivery assembly of any one of claim 1 or claim 2 wherein each of the first branch tubular member, the second branch tubular member and the parent tubular member are a separate sheath.

4. The stent delivery assembly of any one of the previous claims wherein the first branch tubular member and the second branch tubular member are both contained in the parent tubular member.

5. The stent delivery assembly of claim 4 wherein parent tubular member is retractable from the first branch tubular member and the second branch tubular member.

6. The stent delivery assembly of any one of the previous claims wherein the first guidewire lumen comprises a means for moving the first branch tubular member and the second guidewire lumen comprises a means for moving the second branch tubular member.

7. The stent delivery assembly of claim 6 wherein the first guidewire lumen and second guidewire lumen are movable independently of each other.

8. The stent delivery assembly of claim 6 wherein the first guidewire lumen and second guidewire lumen are movable simultaneously.

9. The stent delivery assembly of any one of the previous claims wherein the first branch expandable member and the second branch expandable member are connected to the parent expandable member to form a bifurcated stent.

10. The stent delivery assembly of any one of the previous claims wherein the first branch tubular member and the second branch tubular member and the parent tubular member are contained in an outer tubular member.

11. A stent delivery assembly for treating a lesion at a bifurcation in a body, the bifurcation bifurcating a parent vessel into a first branch vessel and a second branch vessel,
wherein the stent delivery assembly comprises a bifurcated balloon catheter having a parent balloon segment, a first branch balloon segment and a second branch balloon segment, wherein a parent expandable member is carried on the parent balloon segment, a first branch expandable member is carried on the first branch balloon segment and a second branch expandable member is carried on the second branch balloon segment, and wherein the parent balloon segment carrying a parent expandable member, the first branch balloon segment carrying a first branch expandable member and the second branch balloon segment carrying a second branch expandable member are contained in a single tubular member.

12. A method for stenting a bifurcation in a body, the bifurcation bifurcating a parent vessel into a first branch vessel and a second branch vessel, comprising the steps of:
providing a stent delivery assembly comprising;
a first branch tubular member and a second branch tubular member and a parent branch tubular member, the first branch tubular member and the second branch tubular member having a releasable connection between the first branch tubular member and the second branch tubular member, and
a first guidewire lumen and a second guidewire lumen, the first guidewire lumen for receiving a first guidewire and the second guidewire lumen for receiving a second guidewire, the distal end of the first guidewire lumen being connected to the distal end of the first branch tubular member and the distal end of the second guidewire lumen being connected to the distal end of the second branch tubular member,
wherein the stent delivery assembly receives a first branch expandable member in the first branch tubular member, a second branch expandable member in the second branch tubular member and a parent expandable member in the parent tubular member, and
advancing the stent delivery assembly so that the stent delivery assembly is positioned in the parent vessel proximal of the bifurcation,
advancing the first guidewire into the first branch vessel and advancing the second guidewire into the second branch vessel,
advancing the stent delivery assembly until the releasable connection between the first branch tubular member and the second branch tubular member is released by the bifurcation,
advancing the stent delivery assembly so that the first branch expandable member contained in the first branch tubular member enters the first branched vessel and the second branch expandable member contained in the second branch tubular member enters the second branched vessel,
releasing the first branch expandable member in the first branch vessel by advancing the first branch tubular member distally of the first expandable member and releasing the second branch expandable member in the second branch vessel by advancing the second branch tubular member distally of the first expandable member,
releasing the parent expandable member in the parent vessel by withdrawing the parent tubular member proximally of the parent expandable member, and withdrawing the stent delivery assembly.

13. The method of claim 12 wherein the first guide wire lumen comprises a means for advancing the first branch tubular member and the second guidewire lumen comprises a means for advancing the second branch tubular member.

14. The method of claim 13 wherein the first guidewire lumen and second guidewire lumen are movable independently of each other.

15. The method of claim 13 wherein the first guidewire lumen and second guidewire lumen are movable simultaneously.

16. The method of any one of claims 12 to 15 wherein the first branch expandable member contained in a first branch tubular member and the second branch expandable member contained in a second branch tubular member are both contained in the parent tubular member.

17. The method of any one of claims 12 to 16 wherein parent tubular member is retractable from the first branch tubular member and the second branch tubular member prior to advancing a first branch tubular member into a first branch vessel and the second branch tubular member into a second branch vessel.

18. The method of any one of claims 12 to 17 wherein the first branch expandable member and the second branch expandable member are connected to a parent expandable member to form a bifurcated stent.

19. The method of any one of claims 12 to 18 wherein the first branch tubular member and the second branch tubular member and the parent tubular member are contained in an outer tubular member.

20. A method for stenting a bifurcation in a body, the bifurcation bifurcating a parent vessel into a first branch vessel and a second branch vessel, comprising the steps of:
providing a bifurcated balloon catheter having a parent balloon segment for carrying the parent expandable member, a first branch balloon segment for carrying the first branch expandable member and a second branch balloon segment for carrying the second branch expandable member, wherein the parent balloon segment carrying a parent expandable member, the first branch balloon segment carrying a first branch expandable member and the second branch balloon segment carrying a second branch expandable member are contained in a single tubular member, and
after withdrawing the tubular member and exposing the first branch expandable member, the second branch expandable member and the parent expandable member, inflating the balloon catheter segments to expand the first branch expandable member, the second branch expandable member and
the parent expandable member.
